# EUROPEAN PATENT APPLICATION

(11) **EP 1 542 165 A1**
(43) Date of publication of application: **15.06.2005**
(21) Application number: 04257317.0
(22) Date of filing: 25.11.2004
(51) Int. Cl.: G06T 7/00

(54) **Method and apparatus for motion correction in 3-D medical image sequence**

(30) Priority: 28.11.2003 FR 0350927
(71) Applicant: GE Medical Systems Global Technology Company LLC, Waukesha, Wisconsin 53188-1696 (US)
(72) Inventor: Calmon, Guillaumel, 75014 Paris (FR); Lee, Ting-Yim, NG6 4M4 London, Ontario (CA); Drummond, Danielle, Wauwatosa, Wisconsin 53226 (US); Procknow, Karen, Willowbrook, IL 60527 (US)
(74) Representative: Goode, Ian Roy

(57) **Abstract**

In order to prolong the duration of an examination by a scanner the examination is carried out in two phases. A first dynamic phase (101) during which the patient holds his breath and the scanner explores a region Rdyn, and a second delayed phase (102) during which the patient breathes freely and the scanner explores the region Rdel. The latter comprises the region Rdyn. The images acquired during the delayed phase are registered (103-107, 109-113) with relation to at least one dynamic phase image. This registration allows an image sequence covering a long time period for region Rdyn to be obtained. This registration permits being free from the effects of the respiratory and cardiac cycles.

## Description

This invention is directed to a method and apparatus for scanning time series of moving objects. An embodiment of the invention relates to a method and apparatus for producing a volume image sequence of a region of an organ in a living organism. The field of the invention is that of medical imaging and the apparatus allowing image sequences to be obtained from an organ situated within a living organism, the living organism preferably being a human being. In particular, the field of the invention is tomodensitometry, or traditional imaging, as well as_magnetic resonance imaging.

In the prior art, organ examinations are performed with several technologies, including X-ray, tomodensitometry or magnetic resonance, while the patient holds his/her breath. This allows one image sequence on average to be obtained extending over thirty seconds at most. In the prior art, it is of course possible to continue the examination while the patient has begun breathing again, but this is of only low interest, or even no interest. In fact most organs are subject to the influence of the respiratory and cardiac cycles. This is even truer for an organ close to the diaphragm as regards the respiratory cycle. Thus, in the prior art, the images obtained while the patient normally breathes are unusable. In fact, the object of the examination is to obtain an image sequence from a specific region of an organ. The impact of respiration is such that it contributes to the displacement of the organ, which has the direct consequence that the image acquired no longer corresponds to the region that one wishes to observe, or that the image acquired corresponds to the region but that the organ is deformed, or that the image is blurred. In all cases the image sequence is unusable since it is unreadable or not pertinent in terms of localization. This is true to such an extent that image acquisition while patients breathe is not practiced.

In the prior art, it is desired to study the organ and the blood flow characteristics in the organ. This is visualized by injecting a contrast medium in the blood. However, the duration of the examination, corresponding to the period of time during which the patient is able to hold his/her breath, is not sufficient for showing the totality of phenomena produced during a blood cycle. In particular, it is possible to study the arterial phase, but not the venous phase of the blood cycle. In other terms, the prior art is not able to image the cycle of elimination of the contrast medium by an organ.

Numerous methods for extracting information exist. One example is to use a registration between a dynamic image and a delayed image. Image acquisition thus takes place in two successive phases, a dynamic phase during which the patient holds his/her breath and a delayed phase during which the patient breathes freely. In other words, the translation and rotation to apply to a dynamic image is determined so that the image is well integrated with a delayed image. This allows the determination of which information to extract from the delayed image to complete a dynamic image sequence.

In an embodiment of the invention these problems are resolved by a method and apparatus for producing an image sequence extending over a long period and by processing the images acquired while an object, such as a patient, breathes. In this text, unless otherwise indicated, image is understood to refer to volume image. Image acquisition thus can take place in two successive phases, a dynamic phase during which the patient holds his/her breath and a delayed phase during which the patient breathes freely. During the dynamic phase the measuring apparatus produces images from a region R of an organ. Each image comprises a certain number N of fixed sections; this number is fixed according to the construction of the sensor. A section could be considered as an image plane, the juxtaposition of image planes allows a volume image to be constituted. During the dynamic phase, the patient is generally immobile. During the delayed phase the measuring apparatus produces images of a region including the region R. A delayed image thus comprises N + 2P sections. These N + 2P sections are spatially centered on the N sections acquired during the dynamic phase. Displacing the patient with relation to the sensor performs this. The patient thus may be lying down on a mobile table. The displacement of the table is symmetrical with relation to the position of the sensor. Dynamic image is used to refer to an image acquired during the dynamic phase, and delayed image refers to an image acquired during the delayed phase. For the delayed phase the same images are obtained by displacing the sensor with relation to the patient.

Once acquisition has ended, the information issued from the dynamic image is utilized to extract from a delayed image information allowing N sections to be reconstructed. In other terms, the N sections most resembling the N sections from a dynamic image are extracted from the N+2P delayed image sections; these extracted N sections allow an image comparable to a dynamic image to be reconstructed. This reconstruction procedure is repeated for each delayed image acquired during the delayed acquisition phase. Thus an image sequence comprising dynamic images and reconstructed images is obtained.

The invention will be better understood upon reading the following description and examining the accompanying figures, in which:
Figure 1 is an illustration of the steps of a method according to an embodiment of the invention;
Figures 2a and 2b are schematic representations of an apparatus for the implementation of the method according to an embodiment of the invention;
Figure 3 is an illustration of the regions of exploration of the different acquisition phases; and
Figures 4a and 4b are illustrations of the principles of rigid registration.

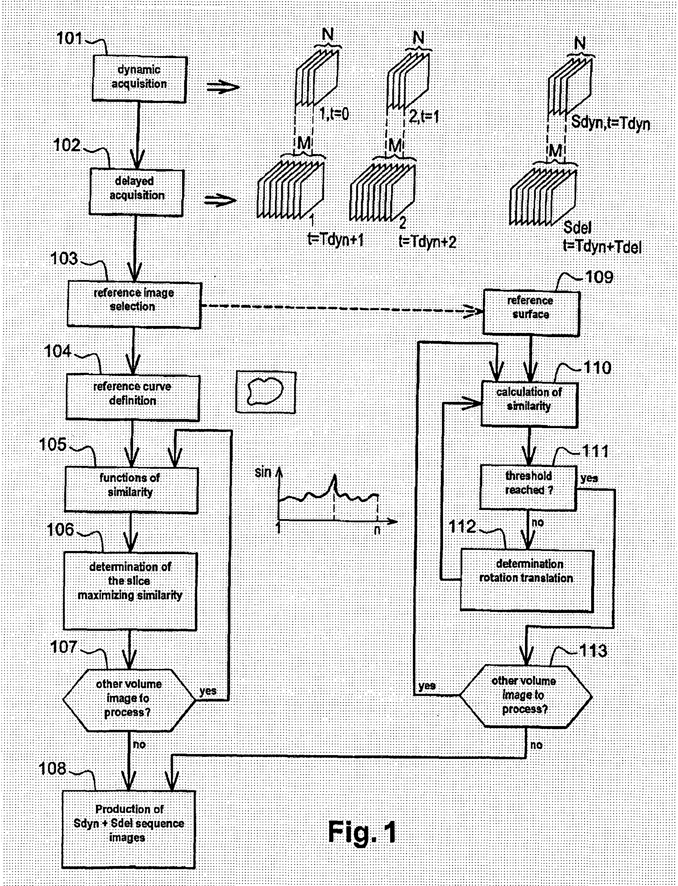

Figure 1 shows a step 101 for dynamic acquisition of volume images from an organ in a living organism. In practice, and for purposes of description, the living organism is a human being. However the invention remains valid with other living organisms, in fact with all higher life forms. Then, adapting the acquisition parameters of the apparatus performing the image acquisition is sufficient.

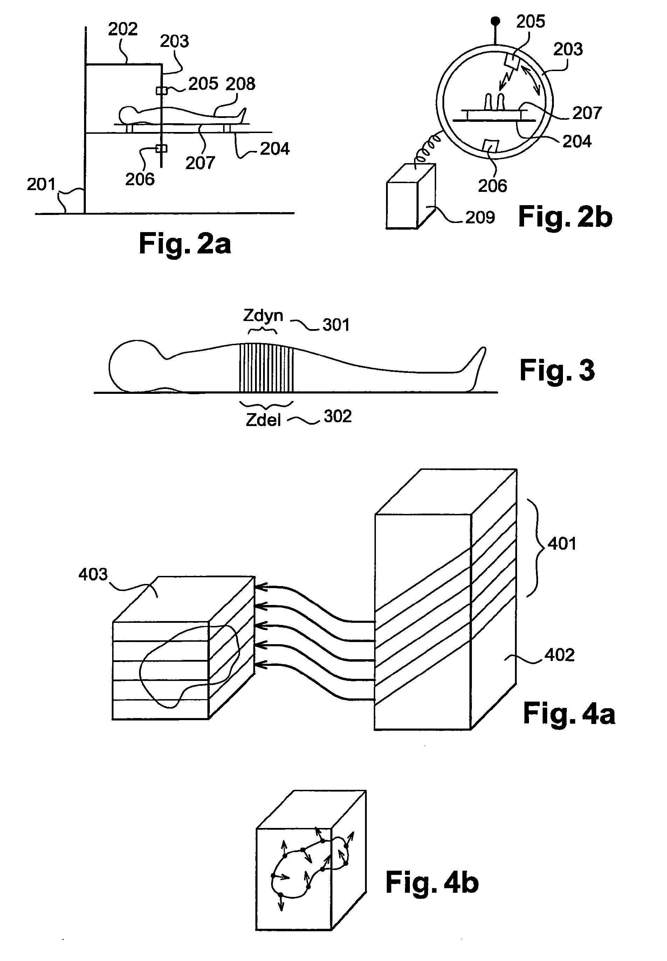

Figure 2a schematically illustrates a volume image acquisition apparatus. In the description a traditional scanner is considered, but in practice this apparatus may be a magnetic resonance apparatus. Figure 2a shows a chassis 201 on which an arm 202 is fixed. The chassis 201 and the arm 202 allow an acquisition device 203 and a horizontal examination table 204 to be supported. The acquisition device 203 is mainly circular, and the examination table 204 is positioned so that a patient placed on the examination table is situated in the center of the device 203. The device 203 comprises an emitter 205 and a sensor 206, the sensor 206 measuring the radiation emitted by the emitter after the said radiation has crossed through the body of the patient and the organ that one wishes to image. The table 204 comprises a platform 207 that is mobile with relation to the chassis 201, and to the device 203. Here it is noted that this is only an example of an image acquisition apparatus. In practice it is possible that it is the sensor that is mobile with relation to the table, and that the table is vertical or inclined.

Figure 2b illustrates that the emitter 205 and the sensor 206 are diametrically opposed. On the various figures, identical references are used for identical elements. During an examination, the emitter 205 and the sensor 206 have a circular movement around the platform 207, and thus around a patient 208 who is lying on this platform. During image acquisition the patient 208 is immobile on the platform 207, and the platform 207 is positioned according to the region of the patient that one wishes to image during the image acquisition.

Figure 2b also shows that the device 203 is connected to a processing device 209. This processing device 209 implements the steps of the invention other than image acquisition. The processing device 209 is either interface with the image acquisition apparatus, or integrated with this apparatus. The processing device thus comprises at least one microprocessor, connection means to the device 203, program and working memories, and a man/machine interface. When an action is automatic, this means that the processing device performs the action with no human intervention required.

The man/machine interface allows the results of the processing according to the invention to be visualized. When an action is attributed to a device, this action is performed by a microprocessor of the device under the control of instruction codes saved in a program memory of the device.

As shown in Figure 1, in step 101 the patient 208 is thus asked by an operator to take his/her place on the platform 207. The operator then positions the platform according to the organ that must be imaged. This organ may be any organ, for example the liver, kidneys, or pancreas. The platform is thus positioned so that a region Rdyn of the patient that one wants to image is situated between the emitter 205 and the sensor 206. Once the positioning has been performed, the patient is asked to hold his breath and the dynamic phase as such starts. The duration Tdyn of this dynamic phase is either predetermined, or associated with the breath holding ability of the patient. The duration of the dynamic phase is thus either arbitrarily predetermined or predetermined by performing a breath-holding test on the patient, or determined by the acquisition apparatus detecting the restart of the patient's respiratory cycle.

In the dynamic phase the image acquisition apparatus is set to acquire images at a certain frequency. The image structure, in particular the number of sections composing an image, as well as the thickness of these sections, is linked to the structure of the sensor. Figure 1 illustrates that the result of the dynamic phase is a series of Sdyn images, each image comprising N sections. In Figure 1 N is equal to four, in practice N is equal to 16, but the invention remains applicable whatever the number of sections of an acquired image. Typically the region Rdyn has a width of 2 cm. This width changes with the type of sensor utilized. The dynamic phase lasts for Tdyn. In practice Tdyn is equal to thirty seconds and rarely exceeds 45 seconds. During Tdyn the apparatus acquires an image at a frequency of one image per second, for example. If the equipment allows this, a higher frequency may be utilized. If the examination does not require such precision, a slower frequency may be utilized. Rapid acquisition frequencies, on the order of a second or less, are usable in the dynamic phase since there is no displacement of the patient along the axis of rotation of the sensor.

in the rest of the description that Tdyn is equal to 30 seconds, and the image acquisition frequency is one image per second. At the end of step 101, the device 203 thus has acquired Sdyn = 30 dynamic images. These are recorded on the processing device 209.

Step 101 proceeds to step 102 for delayed image acquisition. In this delayed phase the platform 207 performs a translation movement along the axis of rotation of the device 203. This translation direction is also orthogonal to the direction of imaging of the device 203. This direction is cyclical and such that a region Rdel including the region Rdyn is imaged during a cycle. Figure 3 illustrates a region Rdyn 301 included in a region Rdel 302. In practice the region Rdel is centered on region Rdyn. The region Rdel comprises M sections with M greater than N, and M = N + 2P. In this case the region Rdel corresponds spatially to the region Rdyn to which P sections have been added on both sides. For example P is equal to N/2. For example the region Rdel extends over 4 cm. This involves a P equal to 8 in the hypothesis or N equal to 16. This extension is variable between others according to the position of the organ imaged with relation to the diaphragm. Symmetry of the region Rdel with rotation to the region Rdyn is not necessary, but the region Rdyn must be included in the region Rdel.

To the extent where there is a displacement of the patient with relation to the sensor, it is not possible to ensure an image acquisition frequency as high as during the dynamic phase. For the delayed phase, the image acquisition frequency is about 1 image every 5 seconds. The delayed phase lasts for Tdel. Tdel may be as long or short as desired. In practice, Tdel is equal to about 2 minutes and 30 seconds. During the delayed phase, the device 203 thus acquires Sdel = 30 images, each of these images comprise M sections.

At the end of step 102, the processing device 209 thus has recorded S = Sdel + Sdyn images. In the example from the description, S is equal to 60 and corresponds to an examination of Tdel + Tdyn = 3 minutes. The period Tdel is thus adjacent to the period Tdyn. This means that the period Tdel immediately follows the period Tdyn. We repeat that nothing limits the duration of the delayed phase, and so that the time does not limit the number of images that may be acquired during this phase.

Step 102 proceeds to step 103 for selecting a reference image, or chosen image. It is from this reference image that the device 209 performs a search for pertinent information in the delayed images. A characteristic of dynamic images is that they are all geo-referenced. That is, if one considers a voxel of one dynamic image, all voxels with the same coordinates in the dynamic images correspond to the same region of the imaged organ. Thus all dynamic images are equivalent from the point of view of a reference to be selected. In practice the first acquired dynamic image is used, but it is possible to perform processing with any acquired dynamic image.

Step 103 proceeds to step 104 for defining a reference curve. This step is performed for each reference image section. A reference curve is, for example, a curve describing the contours of the organ imaged in a section. A reference curve may also be any curve in a section. Preferably the points of the reference curve are in, or near, the imaged organ. This reference curve is either drawn by an operator utilizing the man/machine interface of the device 209 or determined automatically by the device 209 implementing a form recognition algorithm. The reference curve may be identical from one section to another.

Step 104 proceeds to step 105 for determining similarity functions. Previously in this step the device 209 has selected a delayed image that becomes the delayed image being processed. In step 104 the device 209 selects a section of the reference image and calculates a similarity value of this section with each of the delayed image sections being processed. A similarity value measures the resemblance between two sections. The result of this calculation may be represented by a curve having the similarity value on its y-axis and a delayed image section being processed index on its x-axis. These curves are calculated for each reference image section. Thus N similarity curves are obtained.

Step 106 determines the sections of the delayed image being processed that maximize the similarity with the reference image sections. From the similarity curves, the section of the delayed image being processed that maximizes the similarity curve associated with the reference image section is selected for each reference image section. Thus at the end of the selection process, N delayed image sections are obtained allowing an image of N sections called the reconstructed image to be reconstructed. The reconstruction is performed by juxtaposing the selected sections in the order of the reference image sections to which they correspond.

In practice the similarity curves are not necessarily calculated, one just determines, for each reference image section, which delayed image section has the greatest similarity, that is, which section resembles a reference image section the most. The introduction of similarity curves is a variation allowing the selection process to be clearly visualized.

A similarity value between two sections is obtained, for example, by calculating the mutual information existing between the region of interest (ROI) points delimited by the reference curve in a first section, and the analogous ROI in a second section. Another means of obtaining a similarity value between two sections is to calculate the mean deviation between the value of the ROI voxels delimited by the reference curve in a first section, and the value of the analogous ROI voxels in a second section. In the latter case the similarity is all the greater, as the calculated value is small. These values may be calculated for the reference curve, or for arbitrary points, or for all the section points.

In a variation it is possible to displace the delayed image sections, with relation to the reference image sections, to improve the similarity value with the reference image sections. This displacement is then performed in the section plane. This displacement allows a displacement of the organ to be taken into account according to a non-collinear direction to a displacement direction of the platform 207 during the delayed phase. This is then a registration. In this variation several positions relative to the reference image sections are considered with relation to the delayed image sections being processed and the position giving the best similarity value is retained.

Step 106 proceeds to step 107 for determining the number of unprocessed delayed images. In step 107 the device 209 determines if all images acquired during the delayed phase have been processed well. If this is not the case, one processes the following image from among those that have not yet been processed. That is, the delayed image being processed is changed and we pass from step 107 to step 105. This is in fact a classic repeat processing of delayed images so that a reconstructed image is obtained for each delayed image acquired. The delayed images are processed, for example, in the order of their acquisition. In this case the processing of the first delayed images acquired may start before the end of the delayed acquisition phase.

If all delayed images have been processed, step 108 produces a sequence of Sdyn + Sdel images from the region Rdyn of the organ imaged. This sequence is produced by juxtaposing the dynamic images in the order of acquisition, then the reconstructed images in the order of acquisition of the delayed images to which they correspond. Once the sequence is produced, it may be visualized, processed and interpreted via the man/machine interface of the device 209 and/or a processing algorithm.

The device 209 can implement steps 103 to 107.

In a variation step 103 to 107 procedures are performed by an operator. It is this operator who performs the calculation of similarity by visually evaluating the resemblance between the sections.

In another variation we pass from step 103 to step 109 for defining a reference surface. This reference surface is defined in the reference image volume, possibly in several sections of this image. This surface is described by points, each point having coordinates. This surface is preferably included in the organ that is the subject of the examination.

Step 109 proceeds to step 110 for calculating similarity. Previously in step 109 the device 209 has selected a delayed image that is then considered to be the delayed image being processed. In this previous step the device 209 has also positioned the reference image at a starting position in the delayed image being processed. Indeed, it is known that the region Rdyn is included in the region Rdel. It is thus possible to position a dynamic image in a delayed image. Preferably, in the previous step, the reference image is positioned in the center of the image being processed.

In step 110 the device 109 calculates a similarity value between the reference image as it is positioned in the image being processed and the image being processed. This calculation is performed for the points describing the reference surface. For each reference surface point the device 209 also determines in which direction it should displace the point to improve the similarity value. This direction is determined by taking into account the neighborhood of each point defining the reference surface. Such a point is a definition point.

Thus for each definition point a similarity value with all the neighboring points in the image being processed is calculated. The neighboring point allows the best similarity value to be obtained then defining the direction in which the definition point should be displaced to improve the calculated similarity value of the reference image positioned in the image being processed.

At the end of the similarity calculation in step 110 the device 209 is in full knowledge of the similarity value for the reference image positioned in the image being processed. The device 209 also is in full knowledge, for each definition point, of a direction in which it should displace this point to improve the abovementioned similarity value.

Calculating similarity is done by calculating mutual information, a mean deviation between the values of points, or any other methods allowing the resemblance between two images to be evaluated. A direction is, for example, a unit vector. The origin of the vector is the definition point and it extends in the direction of the neighboring point of the definition point allowing the most improvement in the similarity value. In a variation, the direction has a non-unit norm, this norm being then proportional to the improvement caused by the said neighboring point.

Step 110 proceeds to step 111 for evaluating the threshold. There are at least two possible criteria for evaluating the threshold. A first criterion is a predetermined similarity value. Once this value has been reached it has been considered that the position of the reference image maximizing the resemblance with the image being processed has been determined. A second threshold criterion is the maximum number of repetitions for processing a delayed image. A repetition corresponds to a displacement according to step 112. If this repetition threshold is reached it is considered that the resemblance between the reference image and the image being processed cannot be further improved. These two threshold criteria may be combined or used individually.

If the threshold is reached, we pass from step 111 to step 113 for extracting information and determining the next delayed image to process.

If the threshold has not been reached, we pass from step 111 to step 112 for determining a transformation; that is, a translation and a rotation.

In step 112 the device 209 utilizes the directions calculated in step 110 to determine which resulting transformation in design to apply to the reference image in its current position to improve the similarity value. Once determined, the resulting transformation in design is applied to the reference image that then arrives at a new position in the image being processed.

This resulting transformation in design is the result of all the directions associated with the definition points. Thus a translation and a rotation to apply to the reference image is obtained to displace the image in the image being processed. This displacement has the effect of improving the similarity value between the image being processed and the reference image thus displaced. This is illustrated in Figure 4b that shows the reference image comprising a reference surface defined by the definition points. At each definition point is associated a direction represented by a vector. Each vector having a direction relative to its attachment definition point and each vector having a norm. Step 112 proceeds to step 110.

Steps 110 to 112 can be repeated until a threshold is reached. This comes back to displacing the reference image little by little in the image to process, until the position of the reference image that maximizes the similarity value is found; that is, the resemblance between the image being processed and the reference image thus positioned.

The act of determining a resulting transformation in design comes back to determining a displacement. This displacement may also serve as an end-of-repetition criterion. When this displacement becomes less than a predetermined value, then the repetition is interrupted.

In step 113, the device 209 knows the position of the reference image. The device 209 also knows the overall transformation in design that has placed the reference image in this position. This overall transformation in design is the combination of all resulting transformations in design determined in step 112 for the image being processed. The device 209 then extracts from the image being processed the points corresponding to the position of the reference image when the threshold has been reached. Thus an extracted image is obtained to which the device 209 applies the inverse of the rotation of the overall transformation in design to obtain a reconstructed image. Such a reconstructed image is comparable to a reference image since it has the same dimensions, in number of sections, and corresponds to the same region of the imaged organ. This is illustrated in Figure 4a that shows the position of a reference image 401 in a delayed image 402. This position is oblique. From this position, by applying the rotation opposite from the overall transformation in design, a reconstructed image 403 is obtained from the delayed image 402 information and comprises the same number of sections as the reference image 401.

In step 113 the device 209 determines if at least one unprocessed delayed image remains. If not, we pass from step 113 to step 108. If yes the device 209 takes the following delayed image, in the order of acquisition, of the image being processed. This image becomes the new image being processed, and proceeds to step 110.

The variation of steps 109 to 113 implements a rigid registration, that is, the reference surface is not deformed. In practice, it is possible to use a non-rigid registration allowing the reference surface to be deformed. This is utilized for organs undergoing deformation in addition to displacement.

The device 209 implements steps 109 to 113. In a variation, an operator may evaluate a position maximizing the resemblance between a reference image and an image being processed.

The principle of rigid registration that has just been described may also be applied to the variation of steps 105 to 107. In this case instead of considering a volume (image), an image (section) is considered. This then constitutes another variation.

In the variation utilizing rigid registration, the reference curve may be identical for all the delayed images processed, or may be redefined for each delayed image.

An embodiment of the invention allows examinations with the object of producing image sequences to be performed, these examinations having long durations. "Long" is understood to refer to more than thirty seconds, or a duration longer than the normal breath holding ability of a human being.

The examination deals with a long duration allows the extravasation phenomenon to be better-visualized, more pertinent perfusion maps to be established, and more generally allows a better visualization of all phenomena having a long physiological cycle.

All steps 101 to 113 of the process may be performed automatically by the device 209, that is, without operator intervention.

An embodiment of the invention thus provides a method and apparatus for producing a sequence of S volume images from a region Rdyn of an organ in a living organism, each volume image comprising N sections, the process comprising a dynamic acquisition step of Sdyn volume images, this step extending over a time interval Tdyn in the course of which the living organism holds its breath. During the dynamic acquisition phase, the living organism can be immobile on a surface that is itself immobile in relation to the axis of rotation of a sensor.

The process comprises the following step. Delayed acquisition of Sdel volume images from a region Rdel of the organ in a living organism, each volume image comprising M sections, the delayed acquisition extending over a time interval Tdel adjacent to the interval Tdyn, the region Rdel comprising the region Rdyn. Extraction for each volume image of M sections acquired during the delayed acquisition of information elements allowing N sections to be reconstructed, these reconstructed N sections corresponding to a maximum resemblance standard with N sections of a volume image from the dynamic acquisition phase, these reconstructedN sections forming a volume image of the region Rdyn. Production of the sequence of S volume images, where S is equal to Sdyn + Sdel, by juxtaposing the Sdyn acquired volume images and the Sdel reconstructed volume images in the order of their acquisition.

An embodiment of the invention is able to produce image sequences comprising a large number of images. An embodiment of the invention is able to produce image sequences covering a duration longer than the maximum breath holding ability or apnea of the subject object of the examination. An embodiment of the invention is able to produce image sequences covering both the arterial phase and the venous phase of the blood flow in an organ. An embodiment of the invention is able to allow the visualization of the extravasation phenomenon in organs. An embodiment of the invention is able to facilitate the diagnosis of cancer-type diseases. Another object of the invention is to allow a non-invasive examination of an organ.

In an embodiment of the method M is greater than N and the region explored by the M sections are symmetrical with relation to the region explored by the N sections. In an embodiment of the method, during the delayed acquisition step, the living organism is immobile on a mobile surface in translation according to a direction parallel to the axis of rotation of the sensor. In an embodiment of the method, the living organism holds its breath during the dynamic acquisition step, and breathes freely during the delayed acquisition step. In an embodiment of the method, the extraction of information from the volume images acquired during the time interval Tdel is performed by an operator. In an embodiment of the method, the extraction of information from the volume images acquired during the time interval Tdel can be performed automatically.

In an embodiment of the method, to reconstruct a volume image, the following steps are implemented for at least one image acquired during the period Tdel: choice of a reference volume image from among the Sdyn images from the dynamic acquisition step; definition of a reference curve for each chosen image section; comparison of each chosen image section to the M sections of the volume image acquired during the Tdel period so as to determine for these M sections a similarity value in comparison with the chosen image sections, the comparison referring to the coordinate points corresponding to the reference curve; selection, for each chosen image section, of the volume image section acquired during the Tdel period that maximizes the similarity value, the result of the selection being a volume image of N sections.

In an embodiment of the invention, at least the comparison and selection steps are repeated for each volume image acquired during the time interval Tdel.

In an embodiment of the method, the following steps are implemented for at least one image acquired during the delayed phase: choice of a reference image from among the Tdyn images from the dynamic acquisition step; determination of a reference surface in the reference image; calculation of similarity value between the reference image and the image acquired during the delayed phase; evaluation of an end-of-processing threshold for the image acquired during the delayed phase; determination of the rotation/translation that maximizes a similarity function between an image acquired during the Tdel period and the reference image transformed by the rotation translation; and extraction from the volume image acquired during the Tdel period of information corresponding to the volume of the reference image to which the selected rotation/translation has been applied.

In an embodiment of the method, at least the calculation, evaluation and determination steps are repeated for each volume image acquired during the time interval Tdel.

For completeness, various aspects of the invention are set out in the following numbered clauses:
1. A method for producing a sequence of S volume images from a first region Rdyn (301) of an object, each volume image comprising N sections, comprising:
   dynamic acquisition (101) of Sdyn volume images, this step extending over a first time interval Tdyn:
      delayed acquisition (102) of Sdel volume images from a second region (302) Rdel of the object, each volume image comprising M sections, the delayed acquisition extending over a second time interval Tdel adjacent to the first interval Tdyn, the first region Rdel comprising the second region Rdyn;
      extraction (103-107, 103-113) for each volume image of M sections acquired during the delayed acquisition of information elements allowing N sections to be reconstructed, the reconstructed N sections corresponding to a criterion of maximum resemblance with N sections of volume images from the dynamic acquisition phase, the reconstructed N sections forming a volume image of the first region Rdyn; and
      production (108) of the sequence of S volume images, where S is equal to the sum of the dynamic and delayed acquired images (Sdyn + Sdel), by juxtaposing the acquired dynamic (Sdyn) volume images and the reconstructed (Sdel) volume images in the order of their acquisition.
2. The method according to clause 1 wherein M is greater than N and the region explored by the M sections is symmetrical with relation to the region explored by the N sections
3. The method according to one of clauses 1 to 2 wherein during the delayed acquisition, the object is immobile on a mobile surface in translation according to a direction parallel to an axis of rotation of a sensor.
4. The method according to one of clauses 1 to 3 wherein the object holds its breath during the dynamic acquisition, and breathes freely during the delayed acquisition.
5. The method according to one of clauses 1 to 4 wherein the extraction of information from volume images acquired during the second time interval Tdel is performed by an operator.
6. The method according to one of clauses 1 to 5 wherein the extraction of information from volume images acquired during the second time interval Tdel is performed automatically.
7. The method according to one of clauses 1 to 6 wherein reconstruction of a volume image are implemented for at least one image acquired during the second time period Tdel comprising:
   choosing (103) a reference volume image from among the Sdyn images of the dynamic acquisition;
   defining (104) of a reference curve for each section of the chosen image;
   comparison (105) of each chosen image section to the M sections from the volume image acquired during the second time period Tdel so as to determine a similarity value for these M sections with relation to the sections from the chosen image, the comparison being with the coordinate points corresponding to the reference curve; and
   selection (106), for each chosen image section of the volume image section acquired during the second time period Tdel that maximizes the similarity value, the result of the selection being a volume image of N sections.
8. The method according to clause 7 wherein at least the comparison and selection steps are repeated (107) for each volume image acquired during the second time interval Tdel.
9. The method according to one of clauses 1 to 6 wherein implemented for at least one image acquired during the delayed phase comprises:
   choosing (103) of a reference image from among the Tdyn images from the dynamic acquisition;
   determination (109) of a reference surface in the reference image;
   calculation (110) of a similarity value between the reference image and the image acquired during the delayed phase;
   evaluation (111) of an end of processing threshold for the image acquired during the delayed phase;
   determination (112) of a rotation/translation maximizing a similarity function between an image acquired during the period Tdel and the reference image transformed by the rotation translation; and
   extraction (113) from the volume image acquired during the period Tdel of information corresponding to the volume of the reference image to which the selected rotation/translation has been applied.
10. The method according to clause 9 wherein at least the calculation, evaluation and determination steps are repeated for each volume image acquired during the time interval Tdel.
11. An apparatus for producing a sequence of S volume images from a first region Rdyn (301) of an object, each volume image comprising N sections, comprising:
   means (205) for providing a source of emitted radiation;
   means (206) for receiving the emitted radiation after crossing an object placed between the means for providing a source of emitted radiation and the means for receiving the emitted radiation;
   means (209) for processing emitted radiation after crossing an object;
   means (203) for acquiring images of an object based on emitted radiation crossing the object;
   means for dynamic acquisition (101) of Sdyn volume images extending over a first time interval Tdyn:
      means for delayed acquisition (102) of Sdel volume images from a second region (302) Rdel of the object, each volume image comprising M sections, the delayed acquisition extending over a second time interval Tdel adjacent to the first interval Tdyn, the first region Rdel comprising the second region Rdyn;
      means for extraction (103-107, 103-113) for each volume image of M sections acquired during the delayed acquisition of information elements allowing N sections to be reconstructed, the reconstructed N sections corresponding to a criterion of maximum resemblance with N sections of volume images from the dynamic acquisition phase, the reconstructed N sections forming a volume image of the first region Rdyn; and
      means for production (108) of the sequence of S volume images, where S is equal to the sum of the dynamic and delayed acquired images (Sdyn + Sdel), by juxtaposing the acquired dynamic (Sdyn) volume images and the reconstructed (Sdel) volume images in the order of their acquisition.
12. The apparatus according to clause 11 wherein during the delayed acquisition, the object is immobile on a mobile surface in translation according to a direction parallel to an axis of rotation of a sensor.
13. A computer apparatus comprising means for carrying out the steps of clause 1.
14. A computer program comprising code means that when executed on a computer carry out the steps of clause 1.
15. A computer program on a carrier carrying code that when executed on a computer carry out the steps of clause 1.

## Claims

1. A method for producing a sequence of S volume images from a first region Rdyn (301) of an object, each volume image comprising N sections, comprising:
dynamic acquisition (101) of Sdyn volume images, this step extending over a first time interval Tdyn:
delayed acquisition (102) of Sdel volume images from a second region (302) Rdel of the object, each volume image comprising M sections, the delayed acquisition extending over a second time interval Tdel adjacent to the first interval Tdyn, the first region Rdel comprising the second region Rdyn;
extraction (103-107, 103-113) for each volume image of M sections acquired during the delayed acquisition of information elements allowing N sections to be reconstructed, the reconstructed N sections corresponding to a criterion of maximum resemblance with N sections of volume images from the dynamic acquisition phase, the reconstructed N sections forming a volume image of the first region Rdyn; and
production (108) of the sequence of S volume images, where S is equal to the sum of the dynamic and delayed acquired images (Sdyn + Sdel), by juxtaposing the acquired dynamic (Sdyn) volume images and the reconstructed (Sdel) volume images in the order of their acquisition.

2. The method according to claim 1 wherein M is greater than N and the region explored by the M sections is symmetrical with relation to the region explored by the N sections

3. The method according to one of claims 1 to 2 wherein during the delayed acquisition, the object is immobile on a mobile surface in translation according to a direction parallel to an axis of rotation of a sensor.

4. The method according to one of claims 1 to 3 wherein reconstruction of a volume image are implemented for at least one image acquired during the second time period Tdel comprising:
choosing (103) a reference volume image from among the Sdyn images of the dynamic acquisition;
defining (104) of a reference curve for each section of the chosen image;
comparison (105) of each chosen image section to the M sections from the volume image acquired during the second time period Tdel so as to determine a similarity value for these M sections with relation to the sections from the chosen image, the comparison being with the coordinate points corresponding to the reference curve; and
selection (106), for each chosen image section of the volume image section acquired during the second time period Tdel that maximizes the similarity value, the result of the selection being a volume image of N sections.

5. The method according to one of claims 1 to 3 wherein implemented for at least one image acquired during the delayed phase comprises:
choosing (103) of a reference image from among the Tdyn images from the dynamic acquisition;
determination (109) of a reference surface in the reference image;
calculation (110) of a similarity value between the reference image and the image acquired during the delayed phase;
evaluation (111) of an end of processing threshold for the image acquired during the delayed phase;
determination (112) of a rotation/translation maximizing a similarity function between an image acquired during the period Tdel and the reference image transformed by the rotation translation; and
extraction (113) from the volume image acquired during the period Tdel of information corresponding to the volume of the reference image to which the selected rotation/translation has been applied.

6. An apparatus for producing a sequence of S volume images from a first region Rdyn (301) of an object, each volume image comprising N sections, comprising:
means (205) for providing a source of emitted radiation;
means (206) for receiving the emitted radiation after crossing an object placed between the means for providing a source of emitted radiation and the means for receiving the emitted radiation;
means (209) for processing emitted radiation after crossing an object;
means (203) for acquiring images of an object based on emitted radiation crossing the object;
means for dynamic acquisition (101) of Sdyn volume images extending over a first time interval Tdyn:
means for delayed acquisition (102) of Sdel volume images from a second region (302) Rdel of the object, each volume image comprising M sections, the delayed acquisition extending over a second time interval Tdel adjacent to the first interval Tdyn, the first region Rdel comprising the second region Rdyn;
means for extraction (103-107, 103-113) for each volume image of M sections acquired during the delayed acquisition of information elements allowing N sections to be reconstructed, the reconstructed N sections corresponding to a criterion of maximum resemblance with N sections of volume images from the dynamic acquisition phase, the reconstructed N sections forming a volume image of the first region Rdyn; and
means for production (108) of the sequence of S volume images, where S is equal to the sum of the dynamic and delayed acquired images (Sdyn + Sdel), by juxtaposing the acquired dynamic (Sdyn) volume images and the reconstructed (Sdel) volume images in the order of their acquisition.

7. The apparatus according to claim 6 wherein during the delayed acquisition, the object is immobile on a mobile surface in translation according to a direction parallel to an axis of rotation of a sensor.

8. A computer apparatus comprising means for carrying out the steps of claim 1.

9. A computer program comprising code means that when executed on a computer carry out the steps of claim 1.

10. A computer program on a carrier carrying code that when executed on a computer carry out the steps of claim 1.
